# EUROPEAN PATENT APPLICATION

(11) **EP 4 706 649 A1**
(43) Date of publication of application: **11.03.2026**
(21) Application number: 24306448.2
(22) Date of filing: 05.09.2024
(51) Int. Cl.: A61K 31/137, A61K 31/335, A61P 37/08, A61P 9/00, A61P 9/02, A61P 9/04, A61K 9/00

(54) **USE OF ADRENALIN FOR THE TREATMENT OF SHOCKS**

(71) Applicant: BIOPROJET, 75003 Paris (FR)
(72) Inventor: LIGNEAU, Xavier, 35760 SAINT GREGOIRE (FR); LANDAIS, Laurent, 35340 ERCÉ-PRÈS-LIFFRÉ (FR); ROBERT, Philippe, 35740 PACÉ (FR); FINANCE, Olivier, 35000 RENNES (FR); SCHWARTZ, Jean-Charles, 75014 PARIS (FR); LECOMTE, Jeanne-Marie, 75003 PARIS (FR)
(74) Representative: Lavoix

(57) **Abstract**

The present invention concerns novel use of adrenaline for improving the treatment of shocks, comprising the intra-nasal administration of compositions of adrenaline.

## Description

The present invention concerns novel use in the treatment of shocks.

Circulatory shocks, commonly known as "shocks", are life-threatening medical emergencies wherein the organs and tissues of the body are not receiving an adequate flow of blood and thus an adequate level of oxygen. There are three major types of shocks: cardiogenic, hypovolemic, and distributive shocks. Among distributive shocks, the anaphylactic shock and septic shock can be cited.

Among the symptoms of shocks, it can be cited tachycardia, hypotension and signs of poor end-organ perfusion such as low urine output, confusion or weakness.

Among these shocks, the anaphylactic shock can be cited. Anaphylaxis is a severe allergic reaction of rapid onset affecting many body systems and may cause death. It is due to the release of inflammatory mediators and cytokines from mast cells and basophils, typically due to an immunologic reaction but also, sometimes, non-immunologic mechanisms.

In the immunologic mechanism, immunoglobulin E (IgE) binds to an antigen. Antigen-bound IgE then activates FcεRI receptors (Fc epsilon RI receptors) on mast cells and basophils. This leads to release of inflammatory mediators such as histamine. These mediators subsequently increase the contraction of bronchial smooth muscles, trigger vasodilation, increase the leakage of fluid from blood vessels, and cause heart muscle depression.

Non-immunologic mechanisms involve substances that directly cause the degranulation of mast cells and basophils. These include agents such as contrast media, penicillins, opioids, temperature (hot or cold), and vibration.

The prevalence of severe anaphylaxis is high and dramatically increasing each year. For instance, in France, a publication (Monneret-Vautrin, Rev. Fr. Allerg. Immunol. Clin. 2008, 48, 171) quotes a prevalence of 1/10,000 inhabitants and a mortality of 1 per million inhabitants, thereby illustrating the seriousness of the problem.

The balance of evidence from human observations and animal studies suggests that the main pathophysiologic features of anaphylactic shock are a profound reduction in venous tone and fluid extravasation causing reduced venous return (mixed hypovolemic-distributive shock) and depressed myocardial function.

In the occurrence of shocks and more particularly of anaphylactic reactions, an injection of adrenalin (also called epinephrine or adrenaline) within minutes of the onset of symptoms can be lifesaving (Kemp SF et al. Allergy, 2008, 63, 1061-1070). Administration of adrenalin will increase vascular tone, myocardial contractility, and cardiac output in most cases. Adrenalin is a well-known emergency treatment of circulatory shocks such as anaphylactic shock, cardiac arrest, and cardiovascular distress associated with anaphylactic shock, haemorrhagic shock, traumatic shock, infectious shock and secondary shock due to cardiac surgery.

For the treatment of shocks, auto-injectable formulations of adrenalin are commercially available (Anapen^{®}, Epipen^{®}). Adrenalin is typically formulated with sulfites, such as sodium metabisulfite (SMBS) in an aqueous solution for intramuscular injection at a dose of 0.15 mg, 0.3 mg or 0.5 mg (150 µg, 300 µg or 500 µg of adrenalin in 0.3 mL of solution, respectively).

WO2014/053579 discloses the use of a combination of adrenaline and an antidepressant for the treatment of shocks, by intramuscular or subcutaneous injection. These combinations were shown to provide an accelerated bioavailability of adrenalin.

Still, as shocks are a life-threatening condition, there is a constant need to improve the rapidity of onset and/or the overall exposure to the active ingredients for the patient. It is also desirable to improve the shelf-life of the pharmaceutical compositions. It is also desirable to provide a route of administration that allows easy self-administration and full and quick compliance by the patients.

It has now been discovered that the intra-nasal administration of adrenalin, including combination of adrenalin and antidepressant, could improve the treatment of shocks, without excessively increasing dosage of adrenalin in comparison with usual dosing applied for intramuscular route, and contrary to adrenaline formulations described so far for intranasal route.

According to a first object, the present invention concerns a combination comprising:
Adrenalin and
an antidepressant,
for use for intra-nasal administration in the treatment of shocks.

More specifically, the present invention concerns the above combination for use in the treatment of shocks, for which the emergency treatment typically comprises the administration of adrenalin.

The combination for use according to the present invention was shown to potentiate the onset and/or action of adrenalin, thus involving a synergy between its ingredients. Such effects are of great interest as they can be life-saving.

The invention therefore allows the provision of a stable, ready-to-use treatment of shocks with an improved bioavailability in particular in the first ten minutes following the intranasal injection of the combination of adrenalin with an antidepressant.

The combination for use according to the invention is thus of great interest in shocks for which the usual treatment requires the quick administration of adrenalin as it improves the bioavailability of adrenaline.

It is thus an object of the present invention to provide a new use for the treatment for shocks, such as anaphylactic shocks in which the bioavailability and/or efficiency of adrenalin is improved.

### The combinations

The combinations are herein to be understood as actual combined preparations or as kits-of-parts that is as separate components which are meant to be combined, such as for combined administration.

According to an embodiment, said combination may be formulated in the form of a single formulation, or alternatively as a kit, in the form of two or three separate formulations.

Any of these formulations may be in the form of liquid formulations such as aqueous solutions or suspensions, or in the form of solid formulations such as dry-powders, or in the form of gels. Alternative forms of the combination may also be considered.

Any of these formulations may be administered in their actual form or may be extemporaneously compounded into an alternative form before administration.

For example, powder formulations may be extemporaneously compounded into liquid formulations with an aqueous vehicle for nasal administration.

### Adrenalin

Adrenalin (or epinephrine) is both a hormone and a neurotransmitter. It belongs to the group of catecholamines. Adrenalin as used herein refers to the formula: 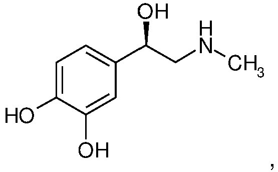 as well as its pharmaceutically acceptable salts.

### The antidepressant

The term « antidepressant » refers to an active principle which is used for the prevention and/or treatment of depression. Depression is a mood disorder characterized by an all-encompassing low mood accompanied by low self-esteem, and by loss of interest or pleasure in normally enjoyable activities.

In one embodiment, the antidepressant is an inhibitor of noradrenalin/monoamine transporters as well as an antagonist of the alpha-1 adrenergic receptors and an antagonist of the H1 receptors.

By "antagonist of the H1 receptor" is meant a compound generally having a Ki inferior to 35 nM for the H1-receptors. By "antagonist of the alpha-1 adrenergic receptor" is meant a compound having a Ki inferior to 200 nM for the alpha-1 adrenergic receptors. By "inhibitor of the noradrenalin/monoamine transporter" is meant a compound having a Ki inferior to 100 nM for the noradrenalin/monoamine transporters.

By "Ki" is meant the dissociation constant obtained by inhibiting the binding of a ligand to a target (e.g., receptor) or the inhibition constant obtained by inhibiting noradrenalin/adrenalin uptake.

The antidepressant can be chosen among the following classes:
- Serotonin-norepinephrine reuptake inhibitors (SNRls),
- Serotonin antagonist and reuptake inhibitors (SARIs),
- Norepinephrine reuptake inhibitors,
- Norepinephrine-dopamine reuptake inhibitors,
- Norepinephrine-dopamine reuptake releasing agents,
- Tricyclic antidepressants, and
- Tetracyclic antidepressants (TeCAs).

In one embodiment, the antidepressant is chosen among the tricyclic antidepressants (TCAs) or one of their pharmaceutically acceptable salts.

TCAs include in particular:
amitriptyline, amoxapine, amitriptylinoxide, butriptyline, clomipramine, demexiptiline, desipramine, dibenzepin, dimetacrine, dosulepin/dothiepin, doxepin, imipramine, imipraminoxide, lofepramine, maprotiline, mianserin, melitracen, metapramine, nitroxazepine, nortriptyline, noxiptiline, pipofezine, propizepine, protriptyline, quinupramine, amineptine (norepinephrine-dopamine reuptake inhibitor), and trimipramine.

In one embodiment, TCAs include:
amitriptyline, amoxapine, amitriptylinoxide, butriptyline, clomipramine, demexiptiline, dibenzepin, dimetacrine, dosulepin/dothiepin, doxepin, imipraminoxide, lofepramine, maprotiline, mianserin, melitracen, metapramine, nitroxazepine, nortriptyline, noxiptiline, pipofezine, propizepine, protriptyline, quinupramine, amineptine (norepinephrine-dopamine reuptake inhibitor), and trimipramine, or one of their pharmaceutically acceptable salts.

In another embodiment, the antidepressant is chosen among the group consisting of amitriptyline, amoxapine, amitriptylinoxide, butriptyline, clomipramine, demexiptiline, dibenzepin, dimetacrine, dosulepin, doxepin, imipraminoxide, lofepramine, maprotiline, mianserin, melitracen, metapramine, nitroxazepine, nortriptyline, noxiptiline, pipofezine, propizepine, protriptyline, quinupramine, amineptine and trimipramine, or one of its pharmaceutically acceptable salts; preferably chosen from amitryptyline, amoxapine, clomipramine, doxepin, imipramine, maprotiline, mianserin nortriptyline, protriptyline and trimipramine. More preferably the antidepressant is chosen among the group consisting of doxepin, trimipramine, amitriptyline and mianserin. More preferably, the antidepressant is chosen among doxepin, imipramine, amitryptiline and their pharmaceutically acceptable salts, preferably doxepin hydrochloride, imipramine hydrochloride or amitryptiline hydrochloride.

According to an embodiment, said antidepressant is doxepin or one of its pharmaceutically acceptable salts.

### The pharmaceutically acceptable salts

The term "pharmaceutically acceptable salts" refers to salts which retain the biological effectiveness and properties of the active ingredient, and which are not biologically or otherwise undesirable. Pharmaceutically acceptable acid addition salts may be prepared from inorganic and organic acids, while pharmaceutically acceptable base addition salts can be prepared from inorganic and organic bases. For a review of pharmaceutically acceptable salts see Berge et al. ((1977) J. Pharm. Sd, vol. 66, 1). For example, the salts include those derived from inorganic acids such as hydrochloric, hydrobromic, sulfuric, sulfamic, phosphoric, nitric, and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, fumaric, methanesulfonic, and toluenesulfonic acid and the like.

The preferred pharmaceutically acceptable salts for the antidepressant are chosen among hydrochloride, mesilate, maleate and fumarate.

The preferred pharmaceutically acceptable salts for doxepin are doxepin hydrochloride salts.

The preferred pharmaceutically acceptable salts for adrenalin are hydrochloride and tartrate salt.

The present invention also encompasses hydrates or hydrated salts or polymorphic crystalline structures, racemates, diastereomers or enantiomers of the above ingredients.

### The additional ingredients

According to an embodiment, the combination may further comprise one or more additional ingredients.

In one embodiment, the combination may comprise one or more pharmaceutically acceptable excipient(s) such as preservative agents, buffers, substances to make the composition isotonic with blood such as sodium chloride, solvents, stabilizers, or antimicrobial preservatives. Pharmaceutically acceptable excipients are well-known to the skilled person. They should not adversely affect the stability, bioavailability, safety, or efficacy of the active ingredients, or cause toxicity or undue local irritation when the composition is administered.

Suitable pharmaceutically excipients for the present invention include in particular salts including sodium chloride, complexing agents such as EDTA, EGTA, alkaline or acidic agents such as NaOH and HCl, respectively, and preservating agents.

In particular, HCl and/or NaOH may be used to ensure the stability of the combination.

According to an embodiment, the combination may comprise a preservative agent.

Said preservative agent may be chosen in particular from antioxidants, such as metabisulfites (MBS) and/or N-acetylcysteine (NAC) and derivatives thereof.

MBS are primarily marketed in the form of sodium metabisulfite (SMBS, E223) or potassium metabisulfite (KMBS, E224). When dissolved in water, these salts release the hydrogen sulfite HSO⁻₃ anion to exert their antioxidant activity.

According to an embodiment, said combination does not comprise MBS, such as SMBS.

According to an alternative embodiment, said combination comprises N-acetylcysteine (NAC) or a derivative thereof as a preservative agent.

NAC is of formula:

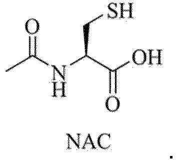

NAC is routinely used in paracetamol intoxication and as a mucolytic agent.

NAC derivatives are known, such as those described in Med.Chem.Comm. 2017 Dec 1; 8(12): 2238-2247. NAC derivatives include in particular N-acetylcysteine amide (NACA) of formula:

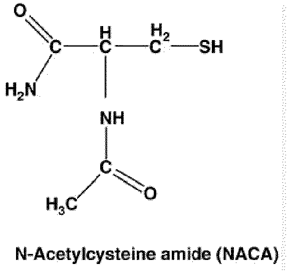

According to an embodiment, when said combination is in the form of a combined preparation i.e. a single formulation, as defined above, it comprises N-acetylcysteine or a derivative thereof as the sole antioxidant i.e. it does not comprise alternative oxidant, in particular it is devoid of metabisulfite (MBS) such as sodium metabisulfite (SMBS).

According to an alternative embodiment, said combination may be in the form of a kit of two or more separate formulations for combined use as defined above, such as a first formulation comprising adrenalin and a second formulation comprising said antidepressant.

According to an embodiment, said first formulation comprises an antioxidant. Typically, said antioxidant may be chosen from N-acetylcysteine or a derivative thereof, ascorbic acid and a bisulfite derivative, such as sodium metabisulfite (SMBS). Preferably, said first formulation comprises N-acetylcysteine or a derivative thereof.

According to an embodiment said second formulation is devoid of an antioxidant or comprises an antioxidant. When it comprises an antioxidant, it may be chosen from N-acetylcysteine or a derivative thereof and/or one or more alternative antioxidant such as ascorbic acid. In particular, said second formulation does not comprise sodium metabisulfite (SMBS).

According to a preferred embodiment, the combination may comprise adrenalin, doxepin, N-acetylcysteine, EDTA, EGTA, sodium chloride and water.

According to a still preferred embodiment, said combination is an aqueous solution consisting of adrenalin, doxepin, N-acetylcysteine, EDTA, EGTA, sodium chloride and water.

### Shocks

By "shock" it is understood a circulatory shock, which may be characterized by a decrease of the organ perfusion. Circulatory shocks are acute and severe pathologies, often life-threatening, and are well-known by physicians.

The term "shock" refers to any shock as defined herein, for which the emergency treatment typically comprises the administration of adrenalin.

In one particular embodiment, the shock is chosen from the group consisting of: anaphylactic shock, cardiac arrest, and cardiovascular distress associated with anaphylactic shock, haemorrhagic shock, traumatic shock, infectious shock and secondary shock due to cardiac surgery. In a particular embodiment, the shock is the anaphylactic shock.

By "treatment", it may be understood the treatment of the causes of the shock and/or the treatment of its symptoms, more particularly the treatment of its symptoms.

The invention also relates to the combination or pharmaceutical composition as defined above, for use in the treatment of shocks.

The invention also relates to a method of treatment of shocks, such as anaphylactic shocks, comprising the administration of a pharmaceutical composition as defined above in a patient in need thereof.

### Pharmaceutical compositions

According to a further object, the present invention also provides a pharmaceutical composition for use for intra-nasal administration in the treatment of shocks, said composition comprising the combination according to the invention, together with one or more pharmaceutically acceptable excipients.

The pharmaceutical composition or combination as defined above is suitable to be delivered into the nasal cavity of a patient.

The delivery means typically depends on the form of said composition or combination.

When it is in the form of a liquid formulation, such as an aqueous solution or suspension, it may be sprayed into the nasal cavity of the patient.

The term "spray" refers to a liquid formulation that is blown or driven through the air in the form of tiny drops. Typically, it refers to a liquid that may be kept in a can or other container, and may be forced out in very small drops.

When it is in the form of a liquid formulation, the volume to be administered is comprised between 0.025 ml and 0.50 ml.

In one embodiment, in a liquid formulation to be sprayed, adrenalin is typically at a concentration comprised between 0.5 mg/ml and 100 mg/ml, preferably between 1 mg/ml and 80.0 mg/ml. In a particular embodiment, adrenalin is at a concentration between 1.5 mg/ml and 20.0 mg/ml. In another embodiment, adrenalin is at a concentration between 2 mg/ml and 10 mg/ml in formulations suitable for adults. In another embodiment, adrenalin is at a concentration between 1 mg/ml and 30 mg/ml in formulations suitable for children.

In another embodiment, in the liquid formulation to be sprayed as defined above, the antidepressant is at a concentration comprised between 0.5 mg/ml and 2000 mg/ml. Preferably, the antidepressant is at a concentration comprised between 1.5 mg/ml and 50 mg/ml.

When the pharmaceutical composition is in a solid form such as a dry-powder, the composition may be delivered with a dry-powder inhaler.

When it is in the form of a dry-powder, the total weight to be administered is comprised between 0.05 and 60 mg.

In an embodiment, in a dry-powder formulation, adrenalin is typically at a dose comprised between 0.05 mg and 12 mg. In a particular embodiment, adrenalin is at a dose between 0.15 mg and 0.6 mg, more preferably between 0.2 mg and 0.6 mg in formulations suitable for adults, or between 0.1 mg and 0.2 mg in formulations suitable for children.

In another embodiment, in a dry-powder formulation, said antidepressant is at a concentration comprised between 0.05 mg and 1200 mg, preferably between 0.15 mg and 12 mg.

According to an embodiment, in a pharmaceutical composition suitable for intra-nasal administration according to the invention, the ratio (in concentration) of adrenalin to the antidepressant (i.e., adrenaline concentration/ antidepressant concentration) may be typically comprised between 1/1 and 1/20, preferably between 1/3 and 1/10.

In another embodiment, the use of the pharmaceutical composition as defined above, comprises administering a dose of adrenalin comprised between 0.05 mg and 6.0 mg. Preferably, the use of the pharmaceutical composition as defined above, comprises administering a dose of adrenalin comprised between 0.05 mg and 0.15 mg for children and between 0.15 mg and 1 mg for adults. More preferably, the use of the pharmaceutical composition as defined above comprises administering a dose of adrenalin of 0.1 to 0.15 mg for children and of 0.3 to 0.5 mg for adults.

In another embodiment, the pharmaceutical composition for use as defined above, comprises administering the antidepressant at a dose comprised between 0.05 mg and 10 mg.

In an embodiment, the combination or pharmaceutical composition is formulated in a dosage form that may be either a unit dosage for single use or a dosage form allowing repeated administrations.

### The devices

In an embodiment, the dosage form is contained in a self-administration device suitable for intra-nasal administration.

The invention thus also relates to a self-administration device suitable for intra-nasal delivery of the pharmaceutical composition of the invention.

According to an embodiment, said self-administration device may be suitable for intra-nasal spraying of a liquid formulation. Accordingly, self-administration devices of the invention include spraying devices.

Alternatively, said self-administration device may be suitable for intra-nasal inhalation of a dry-powder formulation. Accordingly, self-administration devices of the invention may include dry-powder inhalers.

In one embodiment, the self-administration device comprises a vial prefilled with the pharmaceutical composition as defined above. Said vial may be a glass vial prefilled with the pharmaceutical composition as defined above, where said glass vial may be further inserted within an opaque container.

In another embodiment, the self-administration device comprises two vials wherein one first vial is prefilled with a first formulation comprising adrenalin, the second vial is prefilled with a second formulation comprising said antidepressant, and wherein the pharmaceutical composition of the invention is formed by mixing the first and second formulations within the device upon delivery.

This particular embodiment allows the mixing and combined administration of the two formulations at the same time upon delivery.

Typically, this embodiment also allows extemporaneous preparation of a liquid formulation when one the first or second formulation is in the form of a powder and the other formulation is in the form of a liquid formulation.

Typically, the powder formulation is preferred for the less stable ingredient or ingredient that is poorly stable in an aqueous formulation.

By "same time" is meant the time of action of the antidepressant which is the time it takes for the antidepressant to block totally or partially the local vasoconstriction induced by injected adrenalin, therefore accelerating the systemic bioavailability and potentiating the activity of adrenalin

The following examples are presented as particular embodiments of the invention and cannot be considered as a limitation to the present invention.

### FIGURES

Figure 1 illustrates the effects on blood pressure (mean blood pressure is expressed as % change from baseline, and this % change is further integrated over 15 min post dosing) using an intra-nasal administration route for adrenalin and/or doxepin at various doses.
Figure 2 illustrates the effects on blood pressure (mean blood pressure is expressed as % change from baseline, and this % change is further integrated over 30 min post dosing) using intra-nasal (IN) or intra-muscular (IM) administration routes for adrenalin and doxepin.

### EXAMPLES

### 1. In vivo testing

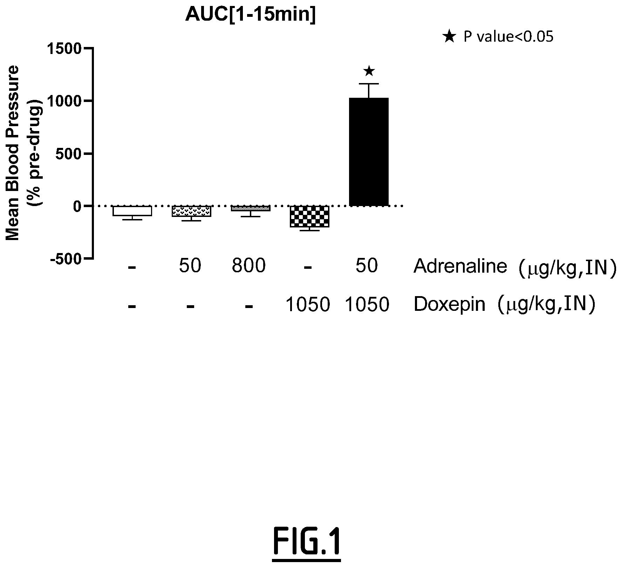

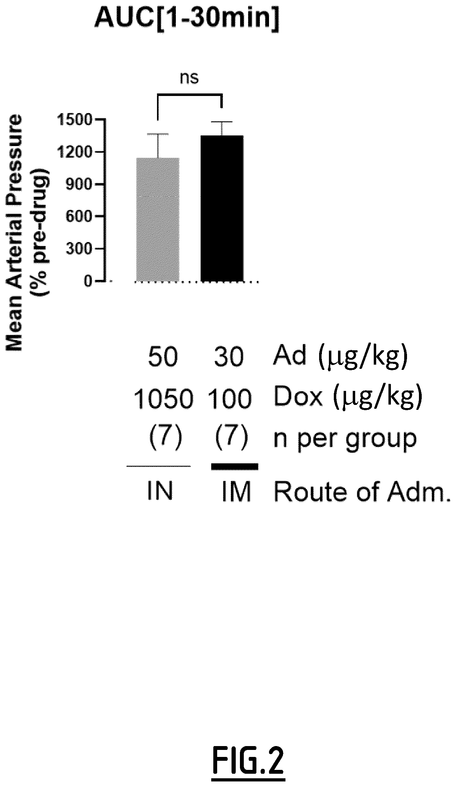

Following pre-medication with non-steroidal anti-inflammatory and analgesic drugs, adult New-Zealand White rabbits, anaesthetized and mechanically ventilated all over the experiment with isoflurane/ O₂/N₂O gas mixture, were catheterized into the carotid artery to measure systemic arterial blood pressure and heart rate parameters. After a stabilization period monitored before drug administration, animals were treated intranasally (IN) with pharmacological entities given both alone, or in combination, by using dedicated nasal pumps delivering 50 µl solution in the form of a spray. For each experimental group, percentages of changes from baseline for mean arterial blood pressure parameter were expressed in Area Under the Curve (AUC) for the first 15-minute period following drug administration (Fig. 1). The hypertensive response of the IN combination, administered at 50 µg/kg adrenaline + 1050 µg/kg doxepin, was further compared to that obtained with intramuscular administration of a 0.3 ml bolus of 30 µg/kg adrenaline + 100 µg/kg doxepin co-administered into the same syringe. Percentages of changes from baseline for mean arterial blood pressure parameter were expressed in AUC for the first 30-minute period following the combination administration (Fig. 2). At the end of the experiment, animals were euthanized with an intravenous overdose of pentobarbital.

### 2. Exemplary Formulations :

### 2.1 Dry-powder formulations

The following dry-powder formulations are considered for intranasal administration (the weight being given for a unit dosage form) :

### • Dry powder 1 :

Adrenalin : 1.7 mg
Doxepin: 5 to 25 mg
NAC: 1 mg

### • Dry powder 2

Adrenalin : 1.7 mg
Doxepin: 5 to 25 mg
MBS: 1.7 mg

The dry-powder formulations may be in the form of a single dry-powder formulation already combining the various ingredients. Alternatively, the formulations may be prepared extemporaneously by mixing the ingredients formulated in dry-powder forms in separate containers.

The dry-powder formulations may be administered with a dry-powder inhaler.

### 2.2 Liquid formulations

### • Liquid formulation 1

Adrenalin : 1.7 mg/mL
Doxepin: 5 to 25 mg/mL
NAC: 1 mg/ml
NaCl : 1.7 mg/mL
Disodium edetate : 0.5 mg/mL
NaOH 1N / HCl 1 N : QS pH= 3.5
Water

### • Liquid formulation 2

Adrenalin : 1.7 mg/mL
Doxepin: 5 to 25 mg/mL
MBS: 1.7 mg/mL
NaCl : 1.7 mg/mL
Disodium edetate : 0.5 mg/mL
NaOH 1N / HCl 1 N : QS pH= 3.5
Water

## Claims

1. A combination comprising:
adrenalin;
an antidepressant, wherein the antidepressant is chosen from the group consisting of amitriptyline, amoxapine, amitriptylinoxide, butriptyline, clomipramine, demexiptiline, dibenzepin, dimetacrine, dosulepin, doxepin, imipraminoxide, lofepramine, maprotiline, mianserin, melitracen, metapramine, nitroxazepine, nortriptyline, noxiptiline, pipofezine, propizepine, protriptyline, quinupramine, amineptine and trimipramine, or one of its pharmaceutically acceptable salts; and
For use for intra-nasal administration in the treatment of shocks.

2. The combination for use according to claim 1, wherein the antidepressant is doxepin or one of its pharmaceutically acceptable salts.

3. The combination for use according to claim 1 or 2, further comprising a preservative agent.

4. The combination for use according to claim 3 wherein said preservative agent is N-acetylcysteine.

5. The combination for use according to claim 4 consisting of adrenalin, doxepin, N-acetylcysteine, EDTA or EGTA, sodium chloride, HCl, NaOH and water.

6. The combination for use according to anyone of claims 1 to 5 where the shock is selected from the group consisting of: anaphylactic shock, cardiac arrest, and cardiovascular distress associated with anaphylactic shock, haemorrhagic shock, traumatic shock, infectious shock and secondary shock due to cardiac surgery.

7. An intra-nasally administrable pharmaceutical composition comprising the combination according to any one of claims 1 to 6 and at least one pharmaceutically acceptable excipient.

8. The pharmaceutical composition according to claim 7, wherein the ratio (in concentration) of adrenalin to the antidepressant (i.e., adrenaline concentration/ antidepressant concentration) is comprised between 1/1 and 1/20.

9. The pharmaceutical composition according to claim 7 or 8 for use for the treatment of shocks by intranasal administration of a dose of adrenalin comprised between 0.05 mg and 6.0 mg and a dose of said antidepressant comprised between between 0.05 mg and 10 mg.

10. An intra-nasal self administration device comprising the combination or pharmaceutical composition according to any one of claims 1 to 9.

11. The intra-nasal spray device according to claim 10 which comprises a vial prefilled with the combination or pharmaceutical composition according to any one of claims 1 to 9.

12. The intra-nasal spray device according to claim 10 which comprises two vials wherein one first vial is prefilled with a first formulation comprising adrenalin, the second vial is prefilled with a second formulation comprising said antidepressant and wherein said pharmaceutical composition according to anyone of claims 7 to 9 is formed by mixing the first and second formulations within the device upon delivery.
